# EUROPEAN PATENT APPLICATION

(11) **EP 2 108 302 A1**
(43) Date of publication of application: **14.10.2009**
(21) Application number: 09005170.7
(22) Date of filing: 08.04.2009
(51) Int. Cl.: A61B 1/01, A61B 17/00

(54) **Endoscopic overtube, treatment instrument and endoscope incorporated into endoscopic overtube, and treatment instrument system including endoscopic overtube**

(30) Priority: 10.04.2008 JP 2008102734
(71) Applicant: Olympus Medical Systems Corporation, Tokyo 151-0072 (JP)
(72) Inventor: Sato, Sunao, Shibuya-ku, Tokyo 151-0072 (JP)
(74) Representative: von Hellfeld, Axel

(57) **Abstract**

An endoscopic overtube 15 of the present invention includes an endoscope insertion passage 15c through which an endoscope 2 is inserted, a treatment instrument insertion passage 15e through which a treatment instrument 17 is inserted, and an ultrasound transmission medium distributor 31 capable of distributing an ultrasound transmission medium 102 from the treatment instrument insertion passage to the endoscope insertion pass age. The thus configured endoscopic overtube of the present invention can hold the ultrasound transmission medium, excel in placing the ultrasound transmission medium, and smoothly supply the ultrasound transmission medium into the treatment instrument insertion passage.

## Description

### BACKGROUND OF THE INVENTION

### 1. Field of the Invention

The present invention relates to an endoscopic overtube, a treatment instrument and an endoscope incorporated into the endoscopic overtube, and a treatment instrument system including the endoscopic overtube, and particularly to the structure of an endoscopic overtube.

### 2. Description of the Related Art

In recent years, studies on surgery technology called NOTES (Natural Orifice Translumenal Endoscopic Surgery) involving inserting an endoscope into an abdominal cavity and drilling a wall of a natural orifice organ to perform surgery on the target organ, for example, perform cholecystectomy, have been actively conducted, and a variety of proposals of endoscopes, treatment instruments, overtubes, and treatment instrument systems including these components used in NOTES have been proposed.

When NOTES is performed, a process of inserting a treatment instrument through a wall of an organ, such as a digestive tract, into which an endoscope has been inserted involves, for example, first puncturing the wall of the organ with a puncture needle, pushing the puncture needle through the wall, performing pneumoperitoneum to form a space outside the organ, and then inserting a suturing instrument, an anastomosing instrument, a T-bar driving instrument, or other treatment instruments through the wall.

In this case, the position where the wall of the organ is punctured with the puncture needle is determined by using an ultrasound endoscope or any other suitable apparatus to monitor the conditions inside and outside the organ, because the conditions outside the organ need to be considered.

A treatment instrument system is used to perform a treatment of this type. An example of the treatment system includes an endoscope and what is called an endoscopic overtube shaped into a hollow tube that covers an insertion portion of the endoscope.

A conventional endoscopic overtube includes an endoscope insertion passage through which an endoscope is inserted and a treatment instrument insertion passage through which a treatment instrument is inserted, and inserting the endoscopic overtube, through which an endoscope or an ultrasound endoscope has been inserted, into an abdominal cavity allows the conditions in the abdominal cavity to be observed in endoscopic images or ultrasound tomographic images.

In an example of such a treatment instrument system, endoscopic image-based and ultrasound tomographic image-based observation is carried out by inserting an ultrasound probe through a treatment instrument channel of an endoscope inserted through an endoscopic overtube. In another example, an ultrasound endoscope, instead of the endoscope, is inserted through the endoscopic overtube.

In ultrasound tomographic image-based observation by using the thus configured conventional treatment instrument system, it is a known fact that the resolution of ultrasound observation images can be improved by filling the gap between a space in the vicinity of an ultrasound transducer provided at the tip of the ultrasound endoscope or the ultrasound probe and an observed site with an ultrasound transmission medium.

In this case, to supply the ultrasound transmission medium to the space in the vicinity of the tip of the ultrasound probe, for example, a water feed conduit provided in the endoscope itself inserted through the endoscopic overtube is used. The space between the distal end portion of the endoscopic overtube and the observed site can thus be filled with the ultrasound transmission medium, whereby sites inside and outside an organ can be observed by using the ultrasound probe in higher-resolution ultrasound tomographic images.

On the other hand, to perform a desired treatment by using a treatment instrument inserted into a treatment instrument insertion passage provided in an endoscopic overtube, the conditions of the distal end portion of the treatment instrument can be observed in ultrasound tomographic images. A variety of such systems have been proposed.

In this case, the position and the conditions of the distal end portion of the treatment instrument, such as a puncture needle, can be observed in high-resolution ultrasound tomographic images by supplying an ultrasound transmission medium to the gap between the vicinity of the distal end portion of the treatment instrument and a desired treatment site through the treatment instrument insertion passage in the endoscopic overtube.

In the conventional treatment instrument system using an endoscopic overtube, however, it is structurally difficult to ensure watertightness around the space between the distal end portion of the endoscopic overtube and the observed site. The thus configured conventional treatment instrument system is therefore problematic in that it is difficult to keep the space filled with an ultrasound transmission medium, which is necessary for high-resolution ultrasound observation.

On the other hand, the diameter of the treatment instrument insertion passage is in general too narrow to supply an ultrasound transmission medium to the distal end portion of the treatment instrument through the treatment instrument insertion passage in the endoscopic overtube. Pressure loss in the treatment instrument insertion passage thus increases, and the ultrasound transmission medium cannot disadvantageously be supplied smoothly to the distal end portion of the treatment instrument.

### SUMMARY OF THE INVENTION

An object of the present invention is to provide an endoscopic overtube capable of not only smoothly supplying an ultrasound transmission medium into the space between the distal end portion of the endoscopic overtube and a site to be observed in such a way that the ultrasound transmission medium stays in the space but also smoothly supplying the ultrasound transmission medium into a treatment instrument insertion passage. Other objects of the present invention are to provide a treatment instrument and an endoscope incorporated into the endoscopic overtube, and a treatment instrument system including the endoscopic overtube.

An endoscopic overtube according to a first aspect of the present invention includes an endoscope insertion passage through which an endoscope is inserted, a treatment instrument insertion passage through which a treatment instrument is inserted, and an ultrasound transmission medium distributor capable of distributing an ultrasound transmission medium from the treatment instrument insertion passage to the endoscope insertion passage.

A second aspect of the present invention provides the endoscopic overtube according to the first aspect of the present invention in which the ultrasound transmission medium distributor is disposed at at least part of an opening of the treatment instrument insertion passage.

A third aspect of the present invention provides the endoscopic overtube according to the first aspect of the present invention in which the ultrasound transmission medium distributor is disposed in the mid-way of the treatment instrument insertion passage.

A fourth aspect of the present invention provides a treatment instrument incorporated into the endoscopic overtube according to the first aspect of the present invention, the treatment instrument including an ultrasound transmission medium flow passage through which an ultrasound transmission medium can flow from a proximal end to a distal end.

A fifth aspect of the present invention provides the treatment instrument according to the fourth aspect of the present invention in which the treatment instrument is a T-bar.

An endoscopic overtube according to a sixth aspect of the present invention includes an endoscope insertion passage through which an endoscope is inserted, a treatment instrument insertion passage through which a treatment instrument is inserted, and at least one of a first protrusion and a second protrusion, the first protrusion provided on an inner peripheral surface of the treatment instrument insertion passage and coming into close contact with an outer peripheral surface of the treatment instrument when the treatment instrument is inserted through the treatment instrument insertion passage, the second protrusion provided on an inner peripheral surface of the endoscope insertion passage and coming into close contact with an outer peripheral surface of the endoscope when the endoscope is inserted through the endoscope insertion passage.

A seventh aspect of the present invention provides a treatment instrument incorporated into an endoscopic overtube including an endoscope insertion passage through which an endoscope is inserted and a treatment instrument insertion passage through which the treatment instrument is inserted. The treatment instrument includes a protrusion disposed on an outer peripheral surface of the treatment instrument, and the protrusion has a shape which comes into close contact with the treatment instrument insertion passage when the treatment instrument is inserted through the treatment instrument insertion passage.

An eighth aspect of the present invention provides an endoscope incorporated into an endoscopic overtube including an endoscope insertion passage through which the endoscope is inserted and a treatment instrument insertion passage through which a treatment instrument is inserted. The endoscope includes a protrusion disposed on an outer peripheral surface of the endoscope, and the protrusion has a shape which comes into close contact with the endoscope insertion passage when the endoscope is inserted into the endoscope insertion passage.

A treatment instrument system according to a ninth aspect of the present invention includes the endoscopic overtube according to the first aspect of the present invention, the treatment instrument according to the fourth aspect of the present invention, the endoscope according to the eighth aspect of the present invention, and an ultrasound probe.

Objects and benefits of the above aspects of the present invention will be more apparent from the following detailed description.

The present invention can provide an endoscopic overtube capable of not only smoothly supplying an ultrasound transmission medium into a space between the distal end portion of the endoscopic overtube and a site to be observed in such a way that the ultrasound transmission medium stays in the space but also smoothly supplying the ultrasound transmission medium into a treatment instrument insertion passage. The present invention can further provide a treatment instrument and an endoscope incorporated into the endoscopic overtube, and a treatment instrument system including the endoscopic overtube.

### BRIEF DESCRIPTION OF THE DRAWINGS

Fig. 1 is a schematic configuration diagram showing an overall configuration of a treatment instrument system according to a first embodiment of the present invention;
Fig. 2 is an enlarged cross-sectional view of a main portion of the distal end portion of an endoscope of an endoscope apparatus and an endoscopic overtube in the treatment instrument system shown in Fig. 1 taken along a plane along an insertion axis direction (the plane along the line [II]-[II] shown in Fig. 3);
Fig. 3 is a front view of the distal end surface of the endoscope and the endoscopic overtube shown in Fig. 2;
Fig. 4 is a view which conceptually shows a state in which a flexible tubular portion of the overtube shown in Fig. 1 is inserted into a luminal organ and a distal end portion of the overtube abuts an observed site;
Fig. 5 is an enlarged longitudinal cross-sectional view of a main portion and shows a cross-section of the vicinity of the distal end portion of an endoscopic overtube in a treatment instrument system according to a second embodiment of the present invention;
Fig. 6 is an enlarged longitudinal cross-sectional view of a main portion and shows a cross-section of a portion in the vicinity of the distal end portion of an endoscopic overtube in a treatment instrument system according to a third embodiment of the present invention;
Fig. 7 is an enlarged longitudinal cross-sectional view of a main portion and shows a cross-section of a portion in the vicinity of the distal end portion of an endoscopic overtube in a variation of the treatment instrument system according to the third embodiment of the present invention; and
Fig. 8 is an enlarged longitudinal cross-sectional view of a main portion and shows a cross-section of an endoscope and a treatment instrument according to a fourth embodiment of the present invention.

### DETAILED DESCRIPTION OF THE PREFERRED EMBODIMENTS

The present invention will be described below with reference to an illustrated embodiment.

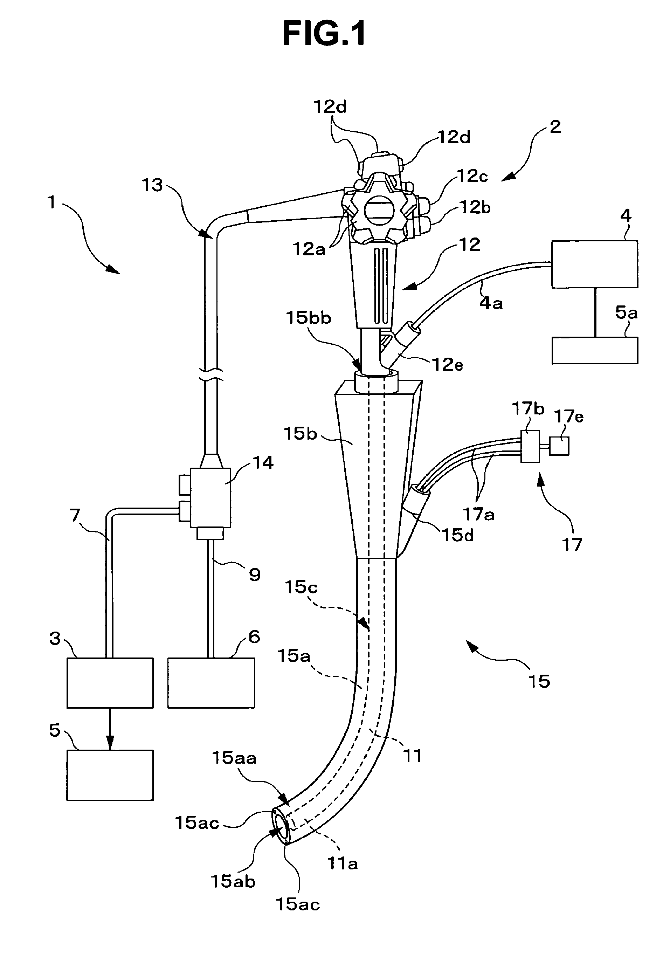

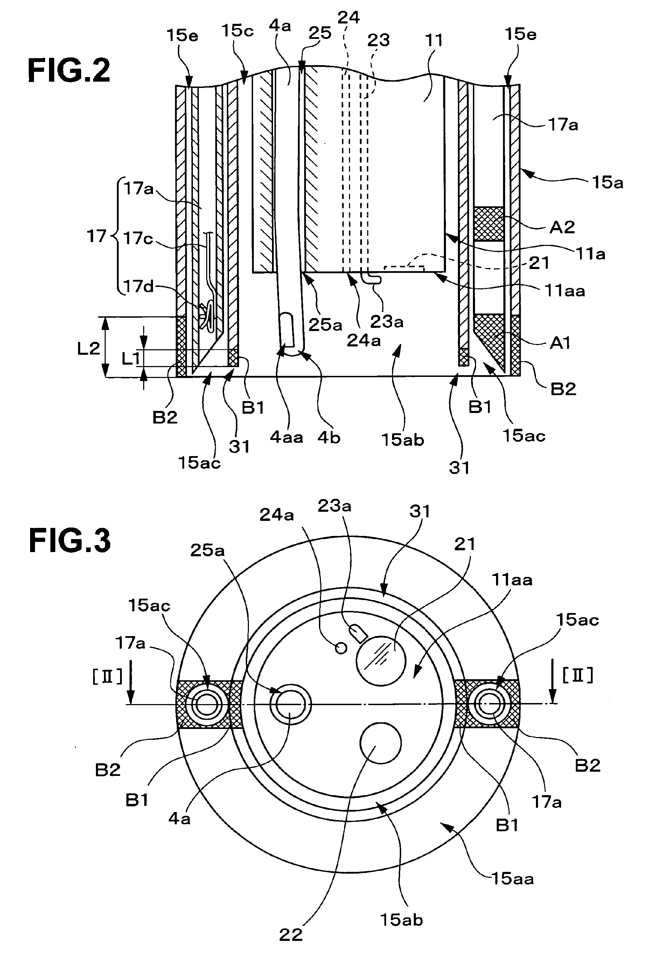

Fig. 1 is a schematic configuration diagram showing an overall configuration of a treatment instrument system according to a first embodiment of the present invention. Fig. 2 is an enlarged cross-sectional view of a main portion of the distal end portion of an endoscope of an endoscope apparatus and an endoscopic overtube in the treatment instrument system shown in Fig. 1 taken along a plane along an insertion axis direction (the plane along the line [II]-[II] shown in Fig. 3). Fig. 3 is a front view of the distal end surface of the endoscope and the endoscopic overtube shown in Fig. 2.

The overall configuration of the treatment instrument system according to the first embodiment of the present invention will first be described below with reference to Figs. 1, 2, and 3.

The treatment instrument system 1 of the present embodiment includes, as shown in Fig. 1, an endoscope apparatus primarily composed of an endoscope 2 having a treatment instrument channel (not illustrated in Fig. 1, see reference numeral 25 in Fig. 2), an endoscopic observation device 3, a display device 5, a light source 6, a video cable 7, and a light source cable 9; an ultrasound observation/measurement device 4 having a blood flow display function and a distance measurement function; an ultrasound probe 4a inserted through the treatment instrument channel in the endoscope 2; a treatment instrument 17 (a suturing instrument, an anastomosing instrument, and a T-bar driving instrument, for example), the distal end portion of which is provided with ultrasound scatterers that scatter ultrasound (not illustrated in Fig. 1, see reference characters A1 and A2 in Fig. 2); and an endoscopic overtube (hereinafter simply referred to as an overtube) 15 having an endoscope insertion passage 15c (see Figs. 1 and 2) through which an insertion portion 11 of the endoscope 2 of the endoscope apparatus can be inserted and treatment instrument insertion passages (not illustrated in Fig. 1, see reference character 15e in Fig. 2) through which the treatment instrument 17 can be inserted, the distal end portion of the endoscopic overtube 15 is provided with ultrasound scatterers that scatter ultrasound (not illustrated in Fig. 1, see reference characters B1 and B2 in Fig. 2).

The endoscope 2 in the endoscope apparatus is primarily composed of an elongated insertion portion 11 to be inserted into the body, an operation portion 12 connected to the proximal end of the insertion portion 11 and used to operate the insertion portion 11, a universal cord 13 extending from a side of the operation portion 12, and a connector 14 provided at an end of the universal cord 13.

The insertion portion 11 is primarily composed of a distal-end rigid portion 11a made of a rigid material, a bending portion connected to the proximal end of the distal-end rigid portion 11a and configured to be bendable in any direction, and a thin, elongated flexible tubular portion, one end of which is connected to the proximal end of the bending portion and the other end of which is connected to the distal end of the operation portion 12. The portions described above are disposed in this order from the distal end side.

An image pickup unit (not particularly illustrated) for endoscopic observation is disposed on the distal end side of the distal-end rigid portion 11a. The image pickup unit includes an observation optical system member, an illumination optical system member, and an image pickup device, and is configured to optically pick up an image of an inner wall surface of a digestive tract or any other luminal organ so as to obtain an image pickup signal that contributes to generation of an image signal for displaying an observational endoscopic image.

To this end, an observation window 21 (indicated by the dotted line in Fig. 2), an illumination window 22 (not illustrated in Fig. 2), and the like are provided in a distal end surface 11aa of the distal-end rigid portion 11a, as shown in Figs. 2 and 3. A water feed nozzle 23a, an air sucking/feeding port 24a, and other components are further formed around the observation window 21 in the distal end surface 11aa.

The water feed nozzle 23a and the air sucking/feeding port 24a are connected to a water feed conduit 23 and an air feed conduit 24, respectively. The water feed conduit 23 and the air feed conduit 24 start from the distal end surface 11aa of the insertion portion 11, pass through the insertion portion 11, the operation portion 12, the universal cord 13, and the connector 14, and reach a water feeder (not particularly illustrated) provided in the endoscopic observation device 3.

The operation portion 12 includes, as shown in Fig. 1, operation members for performing a variety of operations of the endoscope 2, such as angle knobs 12a, which are operation members for freely bending the bending portion of the insertion portion 11 in any direction, upward, downward, rightward, and leftward; an air/water feed button 12b for feeding air and water; a sucking button 12c for performing a sucking operation; and a plurality of operation members 12d for performing a variety of operations, such as switching the display on the display device 5, freezing a displayed image, and releasing.

The operation portion 12 further includes a treatment instrument insertion port 12e (see Fig. 1) protruding from a distal end side of the operation portion 12. The treatment instrument insertion port 12e serves as an insertion port when any of the ultrasound probe 4a and a variety of treatment instruments (not particularly illustrated) is inserted and introduced into the body through the treatment instrument channel 25 (see Fig. 2) in the insertion portion 11 in using the endoscope 2.

The universal cord 13 is a cable that extends from a side of the operation portion 12 as described above and houses a variety of signal lines for transmitting electric signals and other signals, an optical fiber cable for transmitting illumination light, and the like. The connector 14, which connects the endoscope 2 to the endoscopic observation device 3 and the light source 6, is disposed at the distal end portion of the universal cord 13.

The endoscopic observation device 3 is image processing means for generating a video signal for observational endoscopic images by driving and controlling the image pickup device in the image pickup unit in the endoscope 2, receiving an image pickup signal transmitted from the image pickup device, and performing a variety of image processing operations.

The display device 5 receives the video signal produced by the endoscopic observation device 3 and displays corresponding observational endoscopic images.

The light source 6 supplies illumination light to the endoscope 2.

The video cable 7 is a connection cable for electrically connecting the endoscopic observation device 3 to the endoscope 2.

The light source cable 9 is an optical fiber cable composed of a bundle of optical fibers and connecting the light source 6 to the endoscope 2.

A variety of components can be inserted into the treatment instrument insertion port 12e provided in the operation portion 12 of the endoscope 2. The components to be inserted include the ultrasound probe 4a, a distal end portion of which is provided with an ultrasound unit 4aa (not illustrated in Fig. 1, but see Fig. 2) that generates a video signal for ultrasound tomographic image display, and a variety of treatment instruments (not particularly illustrated), such as a drilling instrument, which is an endoscopic needle-type treatment instrument, and a surgical treatment instrument. The treatment instrument insertion port 12e communicates with the treatment instrument channel 25 (see Fig. 2), which extends through the insertion portion 11 to a distal end opening 25a (see Figs. 2 and 3) provided in the distal end surface 11aa of the distal-end rigid portion 11a.

To use the ultrasound probe 4a, it is inserted from the treatment instrument insertion port 12e through the treatment instrument channel 25 in the endoscope 2 as described above. The ultrasound probe 4a has the ultrasound unit 4aa (see Fig. 2) disposed in a distal end portion 4b (see Fig. 2), the ultrasound unit 4aa having a plurality of ultrasound transducers, each of which sends and receives ultrasound, arrayed to form an ultrasound scan surface, and capable of generating an ultrasound signal that contributes to generation of tomographic images (ultrasound tomographic images) of an site inside a body wall. The proximal end of the ultrasound probe 4a is connected to the ultrasound observation/measurement device 4 (see Fig. 1) via a connector. The ultrasound observation/measurement device 4 is connected to a display device 5a for displaying ultrasound tomographic images.

Instead of using the display device 5a, connecting the ultrasound observation/measurement device 4 to the display device 5 with a predetermined connection cable allows the display device 5 to simultaneously display endoscopic images produced by the endoscope 2 and ultrasound tomographic images produced by the ultrasound probe 4a or alternately display the two types of images.

The ultrasound observation/measurement device 4 is an ultrasound tomographic image signal processing device that drives and controls the ultrasound transducers of the ultrasound unit of the ultrasound probe 4a to not only send ultrasound having a predetermined frequency toward an object to be observed but also receive from the ultrasound transducers an electric signal obtained by receiving the ultrasound scattered and reflected off the object being observed, and performs a variety of signal processing operations to generate a video signal for ultrasound tomographic images. The ultrasound observation/measurement device 4 has a blood flow display function and a distance measurement function.

The display device 5a receives the video signal generated by the ultrasound observation/measurement device 4 and displays corresponding observational ultrasound images.

To use the insertion portion 11 of the endoscope 2 described above, it is inserted through the overtube 15. The overtube 15 includes, as shown in Fig. 1, a thin, elongated flexible tubular portion 15a into which the insertion portion 11 of the endoscope 2 is inserted and a proximal end forming portion 15b connected to the proximal end side of the flexible tubular portion 15a.

A large-diameter distal end opening 15ab is formed at a substantially central portion of the distal end, when viewed from the front, of a distal end portion 15aa of the flexible tubular portion 15a and small-diameter treatment instrument insertion passage openings 15ac are formed in portions around the distal end opening 15ab.

The distal end opening 15ab is formed to be slightly larger than the outer diameter of the insertion portion 11 inserted through the overtube 15. A step 31 is formed on the outer peripheral edge of portion the distal end opening 15ab that is at least part of the portion adjacent to the treatment instrument insertion opening 15ac. The step 31 is formed in such a way that the cross-section of the adjacent portion (see Fig. 2) has a concave shape oriented inward in the axial direction from the distal end surface of the overtube 15. That is, the step 31 is provided at the portion in the distal end opening 15ac of the treatment instrument insertion passage 15e that is close to the endoscope insertion passage 15c. The shape of the distal end opening 15ac is partially truncated because the step 31 is formed.

The treatment instrument insertion passage openings 15ac are provided in plurality in the circumferential direction around the distal end opening 15ab. The distal-most end portion of each of the treatment instrument insertion passage openings 15ac communicates with the distal end opening 15ab through the step 31 described above.

In this configuration, when the distal end surface of the overtube 15 abuts a wall or any other part in the body, the step 31 serves as an ultrasound transmission medium distributor that allows the treatment instrument insertion passage 15e to communicate with the endoscope insertion passage 15c so that an ultrasound transmission medium 102 is distributed.

On the other hand, a proximal end opening 15bb is formed in a substantially central portion of the end surface, when viewed from the front, of one end of the proximal end forming portion 15b. The endoscope insertion passage 15c, through which the endoscope 2 (the insertion portion 11 of the endoscope 2) is inserted, is formed between the proximal end opening 15bb and the distal end opening 15ab and passes through the flexible tubular portion 15a and the proximal end forming portion 15b of the overtube 15.

The proximal end forming portion 15b further includes a proximal-end insertion passage opening 15d protruding outward from a side of the proximal end forming portion 15b. The treatment instrument insertion passage 15e (not illustrated in Fig. 1, see Fig. 2), through which a treatment instrument or any other instrument is inserted, is formed between the proximal-end insertion passage opening 15d and the treatment instrument insertion passage opening 15ac in the distal end portion 15aa and passes through the flexible tubular portion 15a and the proximal end forming portion 15b of the overtube 15. That is, the treatment instrument insertion passage 15e is formed from the proximal end to the distal end of the overtube 15.

In the present embodiment, for example, two treatment instrument insertion passages 15e are provided, and the treatment instrument insertion passage openings 15ac of the two treatment instrument insertion passages 15e are symmetrically disposed and angularly spaced apart from each other by 180 degrees, as shown in Fig. 3.

The ultrasound scatterers B1 and B2, which scatter ultrasound, are provided in a predetermined area around each of the treatment instrument insertion passage openings 15ac formed in the distal end surface of the flexible tubular portion 15a of the overtube 15. As shown in Fig. 3, the ultrasound scatterer B1 is provided at the portion around the treatment instrument insertion passage opening 15ac that is close to the distal end opening 15ab of the flexible tubular portion 15a. On the other hand, as shown in Fig. 3, the ultrasound scatterer B2 is provided at the portion around the treatment instrument insertion passage opening 15ac that is on the outer peripheral side of the flexible tubular portion 15a.

In the present embodiment, the ultrasound scatterers B1 and B2 are formed only in the region around each of the treatment instrument insertion passage openings 15ac by way of example. Further, in the present embodiment, when the front side of the distal end portion 15aa of the overtube 15 is viewed, the ultrasound scatterers B1 and B2 are formed over entire outer peripheral surface of each of the treatment instrument insertion passage openings 15ac.

Each of the ultrasound scatterers B1 and B2 is provided in a predetermined area in the direction in which a treatment instrument is inserted through the treatment instrument insertion passage 15e from the treatment instrument insertion passage opening 15ac toward the proximal end, as shown in Fig. 2. In this case, the area of the ultrasound scatterer B1 in the longitudinal direction (insertion direction) is set to be smaller than the area of the ultrasound scatterer B2 in the longitudinal direction.

That is, in Fig. 2, reference character L1 that represents the length of the longitudinal area of the ultrasound scatterer B1 and reference character L2 that represents the length of the longitudinal area of the ultrasound scatterer B2 are set to satisfy the relationship L1<L2.

Each of the treatment instrument insertion passages 15e in the overtube 15 is designed in such a way that a variety of treatment instruments 17 are inserted, for example, a T-bar driving instrument, which is one type of suturing tools. In the present embodiment, Fig. 2 illustrates a case where a T-bar driving instrument is used as the treatment instrument 17.

The T-bar driving instrument 17, which is a suturing instrument, is used as follows: That is, the T-bar driving instrument 17 is inserted from the proximal-end insertion passage opening 15d of the overtube 15 and passes through a plurality of treatment instrument insertion passages 15e (two in the present embodiment), and disposed in such a way that the T-bar driving instrument 17 can freely protrude and retract from the distal end opening 15ab.

The T-bar driving instrument 17 includes flexible, elongated, tubular puncture needles 17a (two in the present embodiment), the tip of each of which is acutely angled and houses suturing tools (a suturing thread 17c, a T-bar 17d, and other components, see Fig. 2); a control box 17b (see Fig. 1) connected to the proximal end of each of the puncture needles 17a, the control box 17b forwarding and retracting the puncture needles 17a and controlling the suturing tools and other components; and a gas/drug supplier 17e (see Fig. 1) connected to the control box 17b, the gas/drug supplier 17e supplying a gas (CO₂: carbon dioxide, for example), a drug, and other substances into the abdominal cavity through the hollow portion of each of the puncture needles 17a.

A plurality of ultrasound scatterers A1 and A2 (two in the present embodiment) that scatter ultrasound are provided on the outer peripheral surface of a distal end portion of each of the puncture needles 17a, the ultrasound scatterers A1 and A2 spaced apart from each other by a predetermined distance in the longitudinal direction of the puncture needle 17a (see Fig. 2). The ultrasound scatterer A1 is provided on the outer peripheral surface of a distal-most end portion of the puncture needle 17a. The ultrasound scatterer A2 is a second ultrasound scatterer spaced apart from the ultrasound scatterer A1 by a predetermined distance. That is, the second ultrasound scatterer A2 serves to roughly check the puncture depth of the corresponding puncture needle 17a. The distance between the ultrasound scatterer A1 and the second ultrasound scatterer A2 is therefore set as appropriate in accordance with the types of treatment and surgery.

In the present embodiment, the ultrasound scatterers A1 and A2 are formed all around the outer peripheral surface of the puncture needle 17a in their respective positions. It is noted that the puncture needle 17a inserted through the treatment instrument insertion passage 15e in the right half of Fig. 2 is not drawn as a cross-sectional view but a side view of the puncture needle 17a to show the side surface thereof.

On the other hand, the proximal-end insertion passage opening 15d of the overtube 15 can be connected to an ultrasound transmission medium supplier (not particularly illustrated) as well as the treatment instrument 17 described above. The ultrasound transmission medium supplier supplies deaerated water, pure water, physiological saline, or any other suitable substance as the ultrasound transmission medium through the treatment instrument insertion passages 15e. In this case, the treatment instrument insertion passages 15e in the overtube 15 serve as ultrasound transmission medium flow passages.

When the thus configured treatment instrument system 1 of the present embodiment is used to perform NOTES-based surgery on an organ in the abdominal cavity, such as cholecystectomy, the insertion portion 11 of the endoscope 2 is inserted through the endoscope insertion passage 15c in the overtube 15, and the distal end surface 11aa of the insertion portion 11 is positioned not to protrude out of the distal end opening 15ab of the overtube 15.

The flexible tubular portion 15a of the overtube 15 in this state is inserted from a natural orifice of a subject (patient) who requires surgery, such as the oral cavity, into a luminal organ of interest, such as the stomach, while the surgeon observes endoscopic images from the endoscope 2. In this case, the endoscope 2 is inserted by using operation members of the operation portion 12, as in a typical endoscopic test using a flexible endoscope.

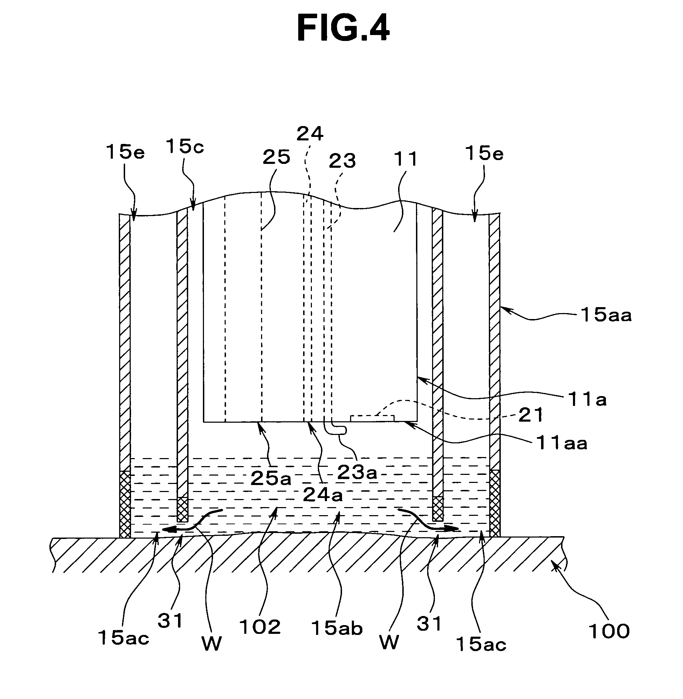

Fig. 4 is a view which conceptually shows a state in which the flexible tubular portion 15a of the overtube 15 is inserted into a luminal organ as described above and the distal end portion 15aa of the overtube 15 abuts an observed site 100.

As shown in Fig. 4, when the distal end portion 15aa of the overtube 15 abuts the observed site 100, the surgeon operates the sucking button 12c on the operation portion 12 of the endoscope 2 to perform sucking. In the sucking operation through the air sucking/feeding port 24a, a predetermined site of the observed site 100 that faces the distal end opening 15ab of the distal end portion 15aa of the overtube 15 is slightly sucked into the distal end opening 15ab. The observed site 100 thus comes into close contact with the end surface of the distal end portion 15aa of the overtube 15 so that a watertight state is created therebetween.

At this point, the surgeon operates the air/water feed button 12b on the operation portion 12 of the endoscope 2 to feed water. The liquid, for example, the ultrasound transmission medium 102, outputted from the water feed nozzle 23a through the water feed conduit 23 in response to the water feed operation stays in the endoscope insertion passage 15c between the interior of the distal end opening 15ab in the distal end portion 15aa of the overtube 15 and the observed site 100, as shown in Fig. 4.

Further, the ultrasound transmission medium 102 flows from the endoscope insertion passage 15c through the steps 31 into the treatment instrument insertion passages 15e, as indicated by the arrows W in Fig. 4. That is, in this case, the steps 31 serve as an ultrasound transmission medium distributor that distributes the ultrasound transmission medium 102 between the treatment instrument insertion passages 15e and the endoscope insertion passage 15c.

Alternatively, in the state shown in Fig. 4, the ultrasound transmission medium 102 can be supplied through the treatment instrument insertion passages 15e by connecting the ultrasound transmission medium supplier (not illustrated) to the proximal-end insertion passage opening 15d (see Fig. 1) of the overtube 15 and performing a predetermined operation to activate the ultrasound transmission medium supplier. In this case, the ultrasound transmission medium 102 flows from the treatment instrument insertion passages 15e through the steps 31, which serve as the ultrasound transmission medium distributor, into the endoscope insertion passage 15c, that is, in the direction opposite to the arrows W in Fig. 4.

As described above, according to the first embodiment, the overtube 15 including the endoscope insertion passage 15c through which the endoscope 2 is inserted and the treatment instrument insertion passages 15e through which the treatment instrument 17 is inserted is configured in such a way that the steps 31, which serve as the ultrasound transmission medium distributor for distributing the ultrasound transmission medium between the treatment instrument insertion passages 15e and the endoscope insertion passage 15c, are provided at at least part of the treatment instrument insertion passage openings 15ac of the treatment instrument insertion passages 15e. The ultrasound transmission medium can therefore be supplied to the treatment instrument insertion passages 15e through the steps 31 (ultrasound transmission medium distributor) by supplying the ultrasound transmission medium into the endoscope insertion passage 15c through the water feed conduit 23 and the water feed nozzle 23a in the endoscope 2.

In the present embodiment, since the steps 31 as the ultrasound transmission medium distributor are provided at at least part of the treatment instrument insertion passage openings 15ac of the treatment instrument insertion passages 15e, the ultrasound transmission medium can be more smoothly distributed between the endoscope insertion passage 15c and the treatment instrument insertion passages 15e.

Alternatively, when the ultrasound transmission medium supplier connected to the proximal-end insertion passage opening 15d of the overtube 15 is used to supply the ultrasound transmission medium into the treatment instrument insertion passages 15e, the ultrasound transmission medium can be supplied into the endoscope insertion passage 15c through the steps 31 (ultrasound transmission medium distributor).

Still alternatively, the ultrasound transmission medium may, of course, be supplied into the endoscope insertion passage 15c through the water feed conduit 23 and the water feed nozzle 23a in the endoscope 2, and at the same time, into the treatment instrument insertion passages 15e from the ultrasound transmission medium supplier. In this case, the amount of ultrasound transmission medium supplied per unit time can be greater than that in the case where only one of the endoscope insertion passage 15c and the treatment instrument insertion passages 15e is used to supply the ultrasound transmission medium, whereby the ultrasound transmission medium can be supplied more efficiently.

Depending on the configuration of the apparatus, the diameter of each of the treatment instrument insertion passages 15e is greater than that of the water feed conduit 23 in the endoscope 2 in some cases. In this case, the amount of ultrasound transmission medium supplied per unit time in the case where the ultrasound transmission medium is supplied through the treatment instrument insertion passages 15e is greater than that in the case where the ultrasound transmission medium is supplied through the water feed conduit 23 in the endoscope into the endoscope insertion passage 15c. Therefore, to supply the ultrasound transmission medium into a space in the vicinity of the distal end portion of the overtube 15, either of the insertion passages may be chosen as appropriate in accordance with the configuration of the apparatus.

Still alternatively, depending on a treatment instrument inserted through the treatment instrument insertion passages 15e in the overtube 15, the ultrasound transmission medium may be supplied through the treatment instrument in combination with the ultrasound transmission medium supplied through the endoscope insertion passage 15c described above. An example of the treatment instrument capable of supplying an ultrasound transmission medium is a hollow puncture needle of a T-bar driving instrument and the like.

According to the present embodiment, since a plurality of ultrasound transmission medium supply paths can be provided and used at the same time, any combination of the plurality of paths may be selected and used as appropriate in accordance with the configuration of the apparatus or the like to be used. More efficient operability is thus achieved.

While in the first embodiment described above, the ultrasound transmission medium distributor in the overtube 15 is composed of the steps 31 provided at at least part of the treatment instrument insertion passage openings 15ac of the treatment instrument insertion passages 15e, the form of the ultrasound transmission medium distributor is not limited thereto.

A second embodiment of the present invention described below shows another form of the ultrasound transmission medium distributor in the overtube.

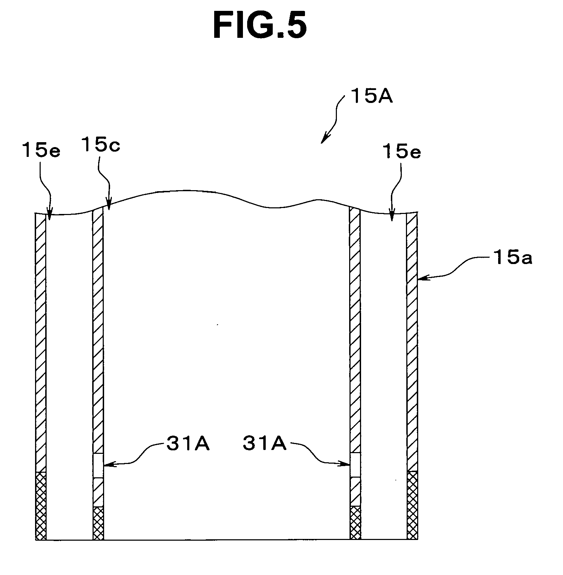

Fig. 5 is an enlarged longitudinal cross-sectional view of a main portion and shows a cross-section of the vicinity of the distal end portion of an endoscopic overtube in a treatment instrument system according to a second embodiment of the present invention. Fig. 5 shows only a distal end portion of the endoscopic overtube, but omits an endoscope, a treatment instrument, and other components to be inserted through the overtube.

The basic configuration of the present embodiment is substantially the same as that of the first embodiment described above. In the present embodiment, the only difference is the form of the ultrasound transmission medium distributor provided in the endoscopic overtube of the treatment instrument system. Therefore, illustration and description of the other configurations will be omitted, and only different portions will be described below.

As shown in Fig. 5, an overtube 15A in the present embodiment has through holes 31A as the ultrasound transmission medium distributor disposed in the mid-way of the treatment instrument insertion passages 15e in the vicinity of the distal end portion, that is, spaced apart from the distal end surface of the overtube 15A by a predetermined distance. The other configurations are the same as those in the first embodiment described above.

In the thus configured overtube 15A, when the ultrasound transmission medium is supplied through the water feed conduit and the water feed nozzle in the endoscope, which is inserted through the endoscope insertion passage 15c, into the endoscope insertion passage 15c as in the first embodiment described above, a predetermined amount of ultrasound transmission medium is accumulated in the space between the site to be observed and the distal end surface of the endoscope insertion passage 15c, and then the ultrasound transmission medium flows into the treatment instrument insertion passages 15e through the through holes 31A.

When the ultrasound transmission medium is alternatively supplied from the ultrasound transmission medium supplier into the treatment instrument insertion passages 15e, a predetermined amount of ultrasound transmission medium is accumulated in the space between the site to be observed and the distal-most end surfaces of the treatment instrument insertion passages 15e, and then the ultrasound transmission medium flows into the endoscope insertion passage 15c through the through holes 31A.

The ultrasound transmission medium can also be supplied into the endoscope insertion passage 15c and the treatment instrument insertion passages 15e at the same time.

As described above, the second embodiment can provide the same advantage as that provided in the first embodiment described above. Further, since the through holes 31A as the ultrasound transmission medium distributor are disposed in the mid-way of the treatment instrument insertion passages 15e, the area of the distal-most end surface of the overtube 15A that comes into close contact with the observed site increases, whereby the watertightness can be more readily enhanced. The above configuration therefore reduces the amount of leak of the ultrasound transmission medium from the space between the distal-most end surface of the overtube 15A and the observed site, and allows the ultrasound transmission medium to be readily held in the space.

In the first and second embodiments described above, there are a variety of conceivable structures of the steps 31 and the through holes 31 A as the ultrasound transmission medium distributor. In addition to the form illustrated in the first embodiment described above (the cutout shape obtained by cutting the distal end surface to form a step at part of the opening) and the hole shape illustrated in the second embodiment described above, conceivable examples include a porous structure and a mesh structure.

The shape of the ultrasound transmission medium distributor can be a circle, an ellipse, a triangle, a rectangle, or other various suitable shapes.

In the treatment instrument systems of the first and second embodiments described above, an observed site that abuts the distal-most end surface of the overtube is sucked by using the air feed conduit and the air sucking/feeding port in the endoscope inserted through the endoscope insertion passage in the overtube, and the distal-most end surface of the overtube comes into close contact with the opposing observed site. A watertight state is thus created between the two portions.

In this case, however, the space between the distal-most end surface of the overtube and the observed site communicates with the outside via the endoscope insertion passage and the treatment instrument insertion passages. It is therefore necessary in some cases to continuously perform the sucking operation described above in order to reliably maintain the watertight state. If the watertight state is not reliably maintained, the ultrasound transmission medium supplied into the space between the distal-most end surface of the overtube and the observed site disadvantageously leaks out of the space.

To address the problem, the watertight state around the space between the distal-most end surface of the overtube and the observed site can be reliably achieved by lowering the pressure in the space through the sucking operation described above. In this state, however, it is conceivable that the flow of the ultrasound transmission medium through the ultrasound transmission medium distributor into the treatment instrument insertion passages is possibly hampered.

A third embodiment of the present invention described below shows another form of the overtube, an improved overtube in which the pressure in the space between the distal-most end surface of the overtube and an observed site is appropriately lowered.

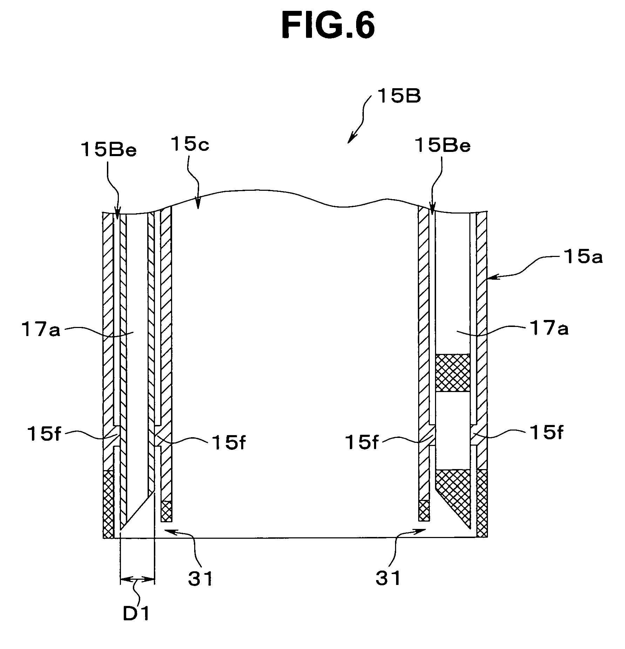

Fig. 6 is an enlarged longitudinal cross-sectional view of a main portion and shows a cross-section of a portion in the vicinity of the distal end portion of an endoscopic overtube in a treatment instrument system according to a third embodiment of the present invention. Fig. 6 illustrates a state in which only a treatment instrument is inserted through the endoscopic overtube.

The basic configuration of the present embodiment is substantially the same as that of the first embodiment described above. In the present embodiment, the only difference is the form of part of each of the treatment instrument insertion passages provided in the endoscopic overtube of the treatment instrument system. Therefore, illustration and description of the other configurations will be omitted, and only different portions will be described below.

As shown in Fig. 6, a treatment instrument insertion passage 15Be in an overtube 15B of the present embodiment has a first protrusion 15f provided on the inner peripheral surface of a portion in the vicinity of the distal end portion of the treatment instrument insertion passage 15Be, the protrusion 15f protruding inward from the inner peripheral surface.

The first protrusion 15f is provided all along the inner peripheral surface of the treatment instrument insertion passage 15Be. When a treatment instrument (a puncture needle 17a of a treatment instrument) is inserted through the treatment instrument insertion passage 15Be, the outer peripheral surface of the puncture needle 17a of the treatment instrument comes into close contact with the first protrusion 15f.

That is, the inner diameter of the first protrusion 15f is set to be substantially equal to the outer diameter of the puncture needle 17a, as indicated by the reference character D1 in Fig. 6. In this case, however, since the puncture needle 17a must smoothly pass through the portion where the first protrusion 15f is disposed, the inner diameter of the first protrusion 15f is set to be slightly greater than the outer diameter of the puncture needle 17a. Further, the cross-sectional shape of the first protrusion 15f may be tapered (not particularly illustrated), for example, in the direction in which the puncture needle 17a is inserted.

The thus formed first protrusion 15f in the treatment instrument insertion passage 15Be, after the puncture needle 17a is inserted through the treatment instrument insertion passage 15Be, can limit the volume in the space in the vicinity of the distal end portion of the treatment instrument insertion passage 15Be. The pressure in the space between the distal-most end surface of the overtube 15B and the observed site can therefore be appropriately lowered, and the watertightness can be maintained more reliably.

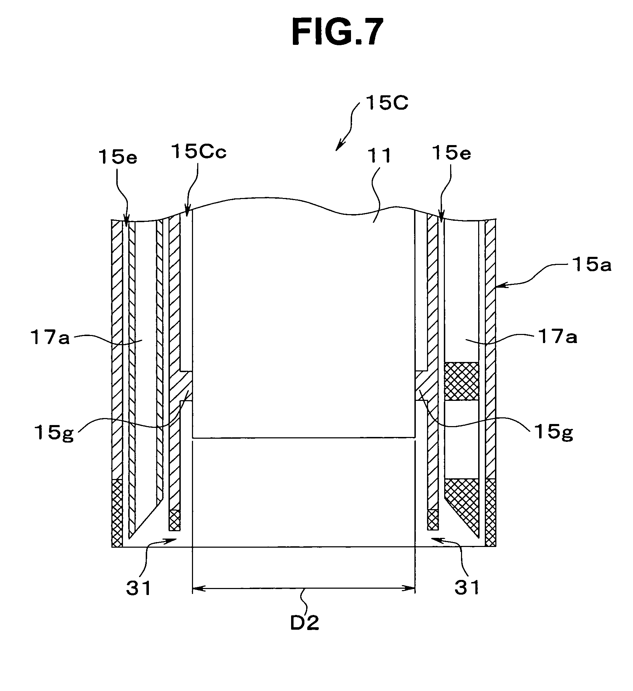

The example shown in Fig. 7 shows a variation of the third embodiment described above, in which the first protrusion is not provided in each of the treatment instrument insertion passages, but the endoscope insertion passage is similarly configured.

That is, Fig. 7 is an enlarged longitudinal cross-sectional view of a main portion and shows a cross-section of a portion in the vicinity of the distal end portion of an endoscopic overtube in a variation of the treatment instrument system according to the third embodiment of the present invention. Fig. 7 shows a state in which an endoscope and a treatment instrument are inserted through the endoscopic overtube.

As described above, an overtube 15C in the present embodiment has a second protrusion 15g provided on the inner peripheral surface of a portion in the vicinity of the distal end portion of an endoscope insertion passage 15Cc, the protrusion 15g protruding inward from the inner peripheral surface.

The second protrusion 15g is provided all along the inner peripheral surface of the endoscope insertion passage 15Cc. When the insertion portion 11 of the endoscope is inserted through the endoscope insertion passage 15Cc, the outer peripheral surface of the insertion portion 11 of the endoscope comes into close contact with the second protrusion 15g.

That is, the inner diameter of the second protrusion 15g is set to be substantially equal to the outer diameter of the insertion portion 11 of the endoscope, as indicated by the reference character D2 in Fig. 7. In this case, however, since the insertion portion 11 of the endoscope must smoothly pass through the portion where the second protrusion 15g is disposed, the inner diameter of the second protrusion 15g is set to be slightly greater than the outer diameter of the insertion portion 11 of the endoscope. Further, the cross-sectional shape of the second protrusion 15g may be tapered (not particularly illustrated), for example, in the direction in which the insertion portion 11 of the endoscope is inserted.

The thus formed second protrusion 15g in the endoscope insertion passage 15Cc, after the insertion portion 11 of the endoscope is inserted through the endoscope insertion passage 15Cc, can limit the volume in the space in the vicinity of the distal end portion of the endoscope insertion passage 15Cc. The pressure in the space between the distal-most end surface of the overtube 15C and an observed site can therefore be appropriately lowered, and the watertightness can be maintained more reliably.

It is noted that the treatment instrument insertion passages in the third embodiment described above may, of course, be combined with the endoscope insertion passage in the variation of the third embodiment to form an overtube.

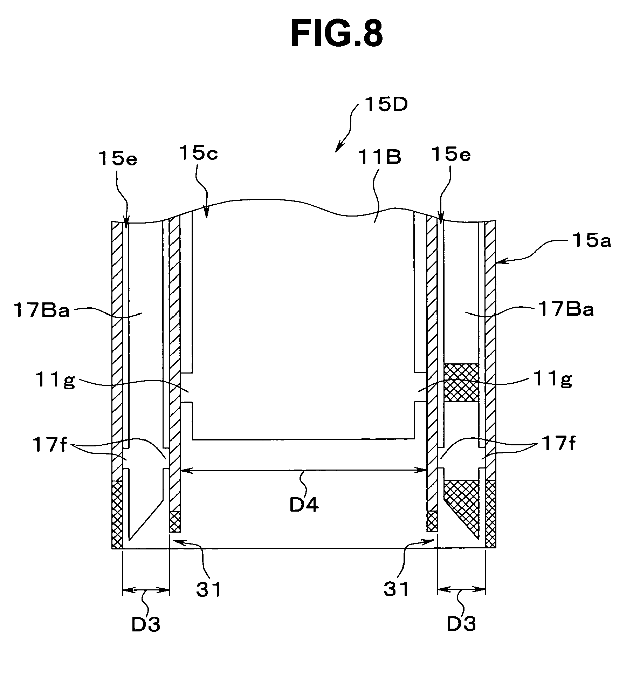

While the third embodiment (Fig. 6) and the variation thereof (Fig. 7) described above show examples of improved shapes of the treatment instrument insertion passage and the endoscope insertion passage in the overtube, improvement can be achieved in other ways. For example, a similar advantage can be provided by improving the shape of a treatment instrument itself inserted through the treatment instrument insertion passages in the overtube or the shape of the endoscope itself inserted through the endoscope insertion passage in the overtube. Fig. 8 shows an exemplary configuration of such an endoscope and treatment instrument.

That is, Fig. 8 is an enlarged longitudinal cross-sectional view of a main portion and shows a cross-section of an endoscope and a treatment instrument according to a fourth embodiment of the present invention. Fig. 8 shows only an enlarged distal end portion of an endoscopic overtube through which the endoscope and the treatment instrument of the present embodiment are inserted.

As shown in Fig. 8, each puncture needle 17Ba of the treatment instrument of the present embodiment has a protrusion 17f provided on the outer peripheral surface of a portion in the vicinity of the distal end portion, the protrusion 17f protruding outward from the outer peripheral surface.

The protrusion 17f is provided all along the outer peripheral surface of the puncture needle 17Ba. When the puncture needles 17Ba are inserted through the treatment instrument insertion passages 15e, the protrusion 17f on each of the puncture needles 17Ba comes into close contact with the inner peripheral surface of the corresponding treatment instrument insertion passage 15e.

That is, the outer diameter of the protrusion 17f of the puncture needle 17Ba is set to be substantially equal to the inner diameter of the treatment instrument insertion passage 15e, as indicated by the reference character D3 in Fig. 8. In this case, however, since the protrusion 17f of the puncture needle 17Ba must smoothly pass through the treatment instrument insertion passage 15e, the outer diameter of the protrusion 17f of the puncture needle 17Ba is set to be slightly smaller than the inner diameter of the treatment instrument insertion passage 15e. Further, the cross-sectional shape of the protrusion 17f may be tapered (not particularly illustrated), for example, in the direction in which the puncture needle 17Ba is inserted.

The thus formed protrusion 17f of the puncture needle 17Ba of the treatment instrument, after the puncture needle 17Ba is inserted through the treatment instrument insertion passage 15e, can limit the volume in the space in the vicinity of the distal end portion of the treatment instrument insertion passage 15e, as in the third embodiment (see Fig. 6) described above. The pressure in the space between the distal-most end surface of the overtube 15D and an observed site can therefore be appropriately lowered, and the watertightness can be maintained more reliably.

On the other hand, as shown in Fig. 8, an insertion portion 11B of the endoscope of the present embodiment has a protrusion 11g provided on the outer peripheral surface of a portion in the vicinity of the distal end portion, the protrusion 11g protruding outward from the outer peripheral surface.

The protrusion 11g is provided all along the outer peripheral surface of the insertion portion 11B of the endoscope. When the insertion portion 11B of the endoscope is inserted through the endoscope insertion passage 15c, the protrusion 11g of the insertion portion 11B of the endoscope comes into close contact with the inner peripheral surface of the endoscope insertion passage 15c.

That is, the inner diameter of the endoscope insertion passage 15c is set to be substantially equal to the outer diameter of the protrusion 11g of the insertion portion 11B of the endoscope, as indicated by the reference character D4 in Fig. 8. In this case, however, since the protrusion 11g of the insertion portion 11B of the endoscope must smoothly pass through the endoscope insertion passage 15c, the outer diameter of the protrusion 11g of the insertion portion 11B of the endoscope is set to be slightly smaller than the inner diameter of the endoscope insertion passage 15c. Further, the cross-sectional shape of the protrusion 11g may be tapered (not particularly illustrated), for example, in the direction in which the insertion portion 11B of the endoscope is inserted.

The thus formed protrusion 11g of the insertion portion 11B of the endoscope, after the insertion portion 11B of the endoscope is inserted through the endoscope insertion passage 15c, can limit the volume in the space in the vicinity of the distal end portion of the endoscope insertion passage 15c, as in the variation of the third embodiment (see Fig. 7) described above. Therefore, the pressure in the space between the distal-most end surface of the overtube 15D and the observed site can be appropriately lowered, and the watertightness can be maintained more reliably.

The treatment instrument and the endoscope in the fourth embodiment described above can provide substantially the same advantage even when only one of the endoscope and the treatment instrument is used to form the treatment instrument system.

While the above embodiments and their variations have been described with reference to the system configured to observe a site in the body in ultrasound tomographic images by inserting the endoscope through the endoscope insertion passage in the endoscopic overtube and using the ultrasound probe inserted through the treatment instrument channel in the endoscope, the system is not necessarily configured as described above. The system may be configured in such a way that an ultrasound endoscope is inserted through the endoscope insertion passage in the endoscopic overtube to observe a site in the body in ultrasound tomographic images.

The present invention is not limited to the embodiments described above, but a variety of changes and applications can of course be made to the extent that they do not depart from the spirit of the present invention. Further, the embodiments described above contain inventions in a variety of stages, and a variety of inventions can be extracted by combining as appropriate a plurality of disclosed configuration requirements. For example, when the problems described in the "BACKGROUND OF THE INVENTION" can be solved and the advantage set forth in the "SUMMARY OF THE INVENTION" is provided even when some of the configuration requirements shown in any one of the embodiments described above are omitted, the configuration without the omitted configuration requirements can be extracted as an invention.

## Claims

1. An endoscopic overtube comprising:
an endoscope insertion passage through which an endoscope is inserted;
a treatment instrument insertion passage through which a treatment instrument is inserted; and
an ultrasound transmission medium distributor capable of distributing an ultrasound transmission medium from the treatment instrument insertion passage to the endoscope insertion passage.

2. The endoscopic overtube according to claim 1,
wherein the ultrasound transmission medium distributor is disposed at at least part of an opening of the treatment instrument insertion passage.

3. The endoscopic overtube according to claim 1,
wherein the ultrasound transmission medium distributor is disposed in the mid-way of the treatment instrument insertion passage.

4. A treatment instrument incorporated into the endoscopic overtube according to claim 1, the treatment instrument comprising an ultrasound transmission medium flow passage through which an ultrasound transmission medium can flow from a proximal end to a distal end.

5. The treatment instrument according to claim 4,
wherein the treatment instrument is a T-bar.

6. An endoscopic overtube comprising:
an endoscope insertion passage through which an endoscope is inserted;
a treatment instrument insertion passage through which a treatment instrument is inserted; and
at least one of a first protrusion and a second protrusion, the first protrusion provided on an inner peripheral surface of the treatment instrument insertion passage and coming into close contact with an outer peripheral surface of the treatment instrument when the treatment instrument is inserted through the treatment instrument insertion passage, the second protrusion provided on an inner peripheral surface of the endoscope insertion passage and coming into close contact with an outer peripheral surface of the endoscope when the endoscope is inserted through the endoscope insertion passage.

7. A treatment instrument incorporated into an endoscopic overtube including an endoscope insertion passage through which an endoscope is inserted and a treatment instrument insertion passage through which the treatment instrument is inserted, the treatment instrument comprising a protrusion disposed on an outer peripheral surface of the treatment instrument,
wherein the protrusion has a shape which comes into close contact with the treatment instrument insertion passage when the treatment instrument is inserted through the treatment instrument insertion passage.

8. An endoscope incorporated into an endoscopic overtube including an endoscope insertion passage through which the endoscope is inserted and a treatment instrument insertion passage through which a treatment instrument is inserted, the endoscope comprising a protrusion disposed on an outer peripheral surface of the endoscope,
wherein the protrusion has a shape which comes into close contact with the endoscope insertion passage when the endoscope is inserted into the endoscope insertion passage.

9. A treatment instrument system comprising:
the endoscopic overtube according to claim 1;
the treatment instrument according to claim 4;
the endoscope according to claim 8; and
an ultrasound probe.
